# EUROPEAN PATENT SPECIFICATION

(11) **EP 3 653 179 B1**
(45) Date of publication and mention of the grant of the patent: **21.06.2023**
(21) Application number: 19383016.3
(22) Date of filing: 18.11.2019
(51) Int. Cl.: A61F 5/02, A61F 5/042, A61F 5/32, A61F 5/37, A61H 1/00, A61H 1/02

(54) **DEVICE FOR THE MANUFACTURE OF A SCOLIOSIS TREATMENT CORSET**
VORRICHTUNG ZUR HERSTELLUNG EINES SKOLIOSE-KORSETTS
DISPOSITIF POUR LA FABRICATION D'UN CORSET POUR LE TRAITEMENT DE LA SCOLIOSE

(30) Priority: 16.11.2018 ES 201831768 U
(43) Date of publication of application: 20.05.2020
(73) Proprietor: Fundación para la Investigación e Innovación Biosanitaria de Asturias (FINBA), 33011 Oviedo (Asturias) (ES); Servicio de Salud del Principado de Asturias (SESPA), 33001 Oviedo (Asturias) (ES)
(72) Inventor: Fidalgo González, José Antonio, 33011 Oviedo (Asturias) (ES); Fernández Suarez, Elena, 33011 Oviedo (Asturias) (ES); Fernández González, Ángel, 33011 Oviedo (Asturias) (ES); Tain Cima, Manuel Tomás, 33011 Oviedo (Asturias) (ES)
(74) Representative: Pons Ariño, Angel

(56) References cited:
- WO-A1-2017/183632
- FR-A1- 3 037 235
- KR-B1- 100 797 812
- US-A- 5 360 392
- US-A1- 2016 193 098

## Description

### OBJECT OF THE INVENTION

The present invention is comprised in the technical field of the orthopedic devices, and relates in particular to a device for the manufacturing of a scoliosis treatment corset.

### BACKGROUND OF THE INVENTION

Scoliosis is defined as a lateral deviation of the spine which, measured in an x-ray, has a Cobb angle greater than 10° and with vertebral rotation. This vertebral rotation is what makes it behave like a structural deformity, as it implies a three-dimensional and irreversible alteration.

Idiopathic scoliosis is the most common form of scoliosis, representing 80-90 %, and develops in a spine with no prior alterations. The etiopathogenesis is still unknown, and for this reason it cannot be prevented, while its diagnosis is by exclusion, i.e., it is only diagnoses when other causes have been ruled out.

Follow-up with patients includes periodic assessment with exams in order to detect a possible progression and, should there be any progression according to defined radiological criteria, to act by trying to change its natural evolution.

Treatment with a corset has proven to be the only treatment capable of changing the natural history of adolescent idiopathic scoliosis as it significantly reduces progression of curvatures, and therefore the indication for surgery on said curvatures. In 1954, Yves Cotrel created a framework, known as Cotrel's frame, in which the corset was manufactured according to the technique of elongation, derotation and lateral flexion (E.D.F. corset).

This technique has been progressively set aside to the detriment of others that imply less physical work and dedication by professionals, as well as better tolerance by patients. On the contrary, new systems for the manufacturing of a corset are more expensive, without there being any significant difference in terms of reducing the scoliosis curvature.

Document US2016193098 discloses a device that enables the patient's body to be maintained in a specific position the corrective pressure forces, that comprise a frame, a central support linked in the lower part of the frame for supporting a user and a mechanical cervical traction element linked to an upper end of the frame for the controlled application of cervical elongation. This document discloses the preamble of claim 1. Documents WO2017183632, KR100797812, US5360392 and FR3037235 disclose devices that comprise a frame, a central support and arms to apply a corrective pressure to the patient.

### DESCRIPTION OF THE INVENTION

The object of the invention consists of a device for the manufacturing of a corset in patients with scoliosis, designed to achieve better tolerance on the part of the user and a better reduction of said scoliosis.

To that end, the device, similar to a table or stretcher, comprises a base or horizontal frame provided with a wide central gap, adjustable central supports, which are telescopically movable in the direction perpendicular to said gap, side corrective or compressing arms, which are also adjustable and movable in the direction transverse to the gap, and a mechanical cervical traction element linked to an upper end of the frame.

The central supports are intended for holding a user in supine decubitus position, and in the preferred embodiment of the device there are three, each of them being independently height-adjustable for better adaptation to the user's morphology and dimensions, for which purpose they have an essentially vertical and telescopic column. These supports are arranged to provide support for the legs, support for the sacrum and another in the upper dorsal region.

The side compressing arms move from the side of the table to exert on the user's torso a corrective pressure for the scoliosis curvature. At their distal end, the compressing arms comprise couplings which adapt to the posterolateral region of the patient's torso. Said pressure is manually adjustable depending on the user's tolerance. Each of the compressing arms can be driven independently with respect to the others and they all incorporate a numerical scale to quantify their movement to the patient's torso.

The mechanical cervical traction element allows a controlled application of cervical elongation. To that end, this element comprises a movable headrest linked to the frame for supporting the user's head. A cable is integrally attached at one of its ends to the headrest, and at the other end to a mechanism exerting a tractive force which moves the cable, and therefore the headrest to which it is integrally linked.

The user's head and the neck, which are joined to the headrest by additional securing elements such as a belt or the like, are subjected to said traction, creating a cervical elongation adjusted by means of an external controller linked to the traction mechanism for determining the tractive force applied, and therefore the cervical elongation exerted in the user.

As for the method for scoliosis treatment which uses the device thus described, it starts by placing the user, in supine decubitus position, in the gap in the frame, held by the central supports. After that the position of the legs and pelvis is adjusted by means of the individual manipulation of each of the corresponding central supports, setting them at the suitable height. This setting allows acting on the alignment of the sagittal plane of the spinal column.

In that moment, with the head supported on the headrest and secured to it by means of the corresponding securing element, elongation of the spinal column can be performed by means of the mechanical cervical traction element.

Then, and once the patient's torso is protected by means of a mesh fabric or similar elements, a glass fibre dressing is placed on the torso. The side compressing arms are rapidly put into place and manual modelling is performed, depending on the type of scoliosis in question. Once the fibre has dried, the traction and the side supports are removed, the patient is taken out of the device and the corset is opened, taking it off the user to then cut and finish it.

The main advantage offered by the device is the above-mentioned tolerance, on the part of the user, to the position and also to the mechanical cervical traction required to be exerted on said user. The result in correcting the scoliosis curvature is also improved.

### DESCRIPTION OF THE DRAWINGS

As a complement to the description provided below, and for the purpose of helping to make the features of the invention more readily understandable, in accordance with a practical preferred embodiment thereof, said description is accompanied by a set of drawings which, by way of illustration and not limitation, represent the following:
Figure 1 shows a perspective view of the device for the manufacturing of a scoliosis treatment corset, in which its main constituent elements can be seen.
Figure 2 shows a detailed top perspective view of the device.

### PREFERRED EMBODIMENT OF THE INVENTION

A detailed explanation of a preferred embodiment of the object of the present invention will now be provided with the aid of figures referred to above.

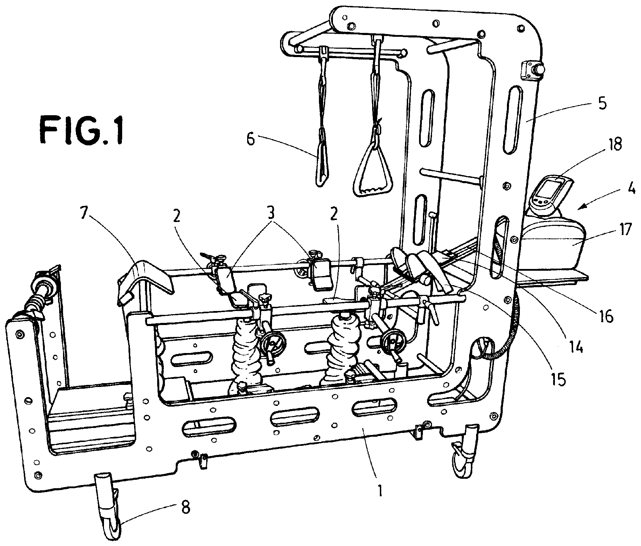

The device for the manufacturing of a scoliosis treatment corset which is described and schematically shown in Figure 1 is fundamentally formed by a base or essentially horizontal frame (1) and provided with a wide central gap, adjustable central supports (2) linked in the lower part to the frame (1) and telescopically movable in the direction perpendicular to said gap, side compressing arms (3) linked on the sides to the frame (1) and also adjustable and movable in the direction transverse to the gap, and a mechanical cervical traction element (4) linked to an upper end of the frame (1).

The preferred embodiment herein described also incorporates vertical prolongations (5) in the form of a column which start out from the top part of the frame (1), at the end where the cervical traction element (4) is located, for holding hand grips (6) hanging on the frame (1), to be gripped by a user's upper extremities. At the opposite end of the frame (1) there is a transverse lower support (7) for supporting the user's lower extremities. The frame (1) is also provided with wheels (8) to facilitates its movement by rolling.

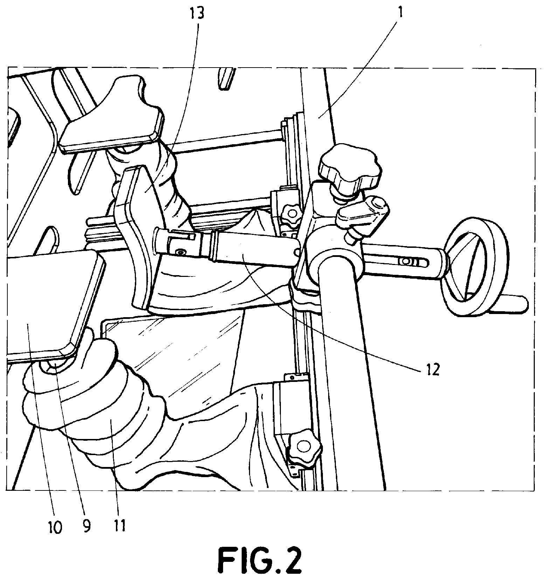

The central supports (2), shown in greater detail in Figure 2, are three in number in this embodiment and serve for supporting and adjusting the position of the user's legs, sacrum and upper dorsal region, respectively, which allows acting on the alignment in the sagittal plane of the spinal column.

Each of said central supports (2), the height of which is independently adjustable, comprises a telescopic column (9), which is essentially vertical and longitudinally movable in the upward and downward directions. The telescopic column (9), the drive of which is hydraulic in this preferred embodiment, has a lower end integrally linked to the frame (1) and is finished at its upper end with a tray (10) where the user's corresponding anatomical area is supported. In this preferred embodiment, the telescopic columns (9) are covered with corresponding flexible sheaths (11).

In the embodiment herein described, the device comprises four compressing arms (3), arranged facing one another two-by-two and linked to two opposite sides of the frame (1), for being coupled with the user's torso and for the controlled and adjustable application of a corrective pressure for the scoliosis. Said compressing arms (3) are independently movable, in the direction transverse to the gap, where the user is located, held by the central supports (2).

Each of the compressing arms (3) comprises a screw (12) that is transverse to the side of the frame (1) where it is located, said screw (12) being linearly movable. The screw (12) is finished at its end with a flat bar-type coupling (13), intended for being in contact with the user's torso for the application of pressure, for which purpose it has a curved geometry which improves the anatomical adaptation and tolerance.

The mechanical cervical traction element (4) allows the controlled application of cervical elongation. To that end, this element comprises a headrest (14) linked to the frame (1) for supporting the user's head, said headrest (14) being provided with additional securing elements (15), consisting in this case of an adjustable belt. A cable (16) is integrally linked at one of its ends to the headrest (14), and at the other end to a traction mechanism (17) for moving the cable (16), and therefore the headrest (14) to which it is integrally linked. Said cervical traction element (4) also comprises a controller (18) linked to the traction mechanism (17) for determining the tractive force applied in the cable (16), and therefore the cervical elongation exerted in the user.

## Claims

1. A device for the manufacturing of a scoliosis treatment corset comprising:
- a essentially horizontal frame (1) provided with a wide central gap, wherein the user is intended to be placed in said central gap;
- central supports (2) linked in the lower part to the frame (1) for supporting a user;
- a mechanical cervical traction element (4) linked to an upper end of the frame (1) for the controlled application of cervical elongation;
the device being **characterised in that** it further comprises side compressing arms, linked on the sides to the frame to apply a corrective pressure for the scoliosis and **in that** each of the central supports (2) comprises:
- a telescopic column (9), which is essentially vertical and longitudinally movable in the upward and downward directions, said telescopic column (9) having a lower end integrally linked to the frame (1), and;
- a tray (10) linked to an upper end of the telescopic column (9), for supporting the user.

2. The device for the manufacturing of a scoliosis treatment corset according to claim 1, **characterised in that** the telescopic column (9) has a hydraulic-type drive.

3. The device for the manufacturing of a scoliosis treatment corset according to claim 1, **characterised in that** each of the compressing arms (3) in turn comprises:
- a screw (12) that is transversely movable with respect to the frame (1), and
- a flat bar-type coupling (13) linked to a free end of the screw (12), intended for being in contact with the user's torso.

4. The device for the manufacturing of a scoliosis treatment corset according to claim 2, **characterized in that** the flat bar-type coupling (13) has a curved geometry for adapting to the user's anatomy.

5. The device for the manufacturing of a scoliosis treatment corset according to claim 1, **characterised in that** the mechanical cervical traction element (4) comprises:
- a headrest (14) linked to the frame (1) for supporting the user's head,
- additional securing elements (15) linked to the headrest (14) for immobilising the head,
- a cable (16) integrally linked at one end to the headrest (14),
- a traction mechanism (17) integrally linked to the other end of the cable (16) for executing a tractive force, and
- a controller (18) linked to the traction mechanism (17) for determining the tractive force applied, and therefore the cervical elongation exerted in the user.

6. The device for the manufacturing of a scoliosis treatment corset according to any of the preceding claims, **characterised in that** the side compressing arms (3) comprise a distal end where are arranged couplings configured for being adapted to the posterolateral region of the patient's torso.

7. The device for the manufacturing of a scoliosis treatment corset according to any of the preceding claims, **characterised in that** each of the side compressing arms (3) can be driven independently with respect to the others.

## Patentansprüche

1. Vorrichtung zur Herstellung eines Skoliose-Korsetts, das Folgendes umfasst:
- einen im Wesentlichen horizontalen Rahmen (1) mit einer breiten zentralen Lücke, wobei der Benutzer in der zentralen Lücke platziert werden soll;
- zentrale Stützen (2), die zum Stützen eines Benutzers im unteren Teil des Rahmens (1) verbunden sind;
- ein mechanisches HWS-Traktionselement (4), das zur gesteuerten Anwendung einer HWS-Dehnung mit einem oberen Ende des Rahmens (1) verbunden ist;
wobei die Vorrichtung **dadurch gekennzeichnet ist, dass** sie ferner seitliche Kompressionsarme umfasst, die an den Seiten mit dem Rahmen verbunden sind, um einen Korrekturdruck für die Skoliose auszuüben, und dadurch, dass jede der zentralen Stützen (2) Folgendes umfasst:
- eine Teleskopsäule (9), die im Wesentlichen vertikal und längs in die Aufwärts- und die Abwärtsrichtung bewegbar ist, wobei die Teleskopsäule (9) ein unteres Ende aufweist, das einstückig mit dem Rahmen (1) verbunden ist, und;
- eine Platte (10), die zum Stützen des Benutzers mit einem oberen Ende der Teleskopsäule (9) verbunden ist.

2. Vorrichtung zur Herstellung eines Skoliose-Korsetts nach Anspruch 1, **dadurch gekennzeichnet, dass** die Teleskopsäule (9) einen hydraulischen Antrieb aufweist.

3. Vorrichtung zur Herstellung eines Skoliose-Korsetts nach Anspruch 1, **dadurch gekennzeichnet, dass** jeder der Komprimierungsarme (3) wiederum Folgendes umfasst:
- eine Schraube (12), die mit Bezug auf den Rahmen (1) quer bewegbar ist, und
- eine flache Stabkupplung (13), die mit einem freien Ende der Schraube (12) verbunden und für einen Kontakt mit dem Oberkörper des Benutzers vorgesehen ist.

4. Vorrichtung zur Herstellung eines Skoliose-Korsetts nach Anspruch 2, **dadurch gekennzeichnet, dass** die flache Stabkupplung (13) zum Anpassen an die Anatomie des Benutzers eine gekrümmte Geometrie aufweist.

5. Vorrichtung zur Herstellung eines Skoliose-Korsetts nach Anspruch 1, **dadurch gekennzeichnet, dass** das mechanische HWS-Traktionselement (4) Folgendes umfasst:
- eine Kopfstütze (14), die zum Stützen des Kopfes des Benutzers mit dem Rahmen (1) verbunden ist,
- zusätzliche Sicherungselemente (15), die zum Ruhigstellen des Kopfes mit der Kopfstütze (14) verbunden sind,
- ein Kabel (16), das an einem Ende einstückig mit der Kopfstütze (14) verbunden ist,
- einen Traktionsmechanismus (17), der zum Ausführen einer Traktionskraft einstückig mit dem anderen Ende des Kabels (16) verbunden ist, und
- eine Steuerung (18), die zum Bestimmen der ausgeübten Traktionskraft und somit der im Benutzer ausgeübten HWS-Dehnung mit dem Traktionsmechanismus (17) verbunden ist.

6. Vorrichtung zur Herstellung eines Skoliose-Korsetts nach einem der vorhergehenden Ansprüche, **dadurch gekennzeichnet, dass** die seitlichen Kompressionsarme (3) ein distales Ende umfassen, an dem Kupplungen angeordnet sind, die zum Anpassen einer posterolateralen Region des Oberkörpers des Patienten ausgelegt sind.

7. Vorrichtung zur Herstellung eines Skoliose-Korsetts nach einem der vorhergehenden Ansprüche, **dadurch gekennzeichnet, dass** jeder der seitlichen Kompressionsarme (3) mit Bezug auf die anderen unabhängig angetrieben werden kann.

## Revendications

1. Dispositif pour la fabrication d'un corset de traitement de scoliose comprenant :
- un châssis essentiellement horizontal (1) pourvu d'un large espace central, dans lequel l'utilisateur est destiné à être placé dans ledit espace central ;
- des supports centraux (2) liés dans la partie inférieure au châssis (1) pour supporter un utilisateur ;
- un élément mécanique de traction cervicale (4) lié à une extrémité supérieure du châssis (1) pour l'application contrôlée d'un allongement cervical ;
le dispositif étant **caractérisé en ce qu'**il comprend en outre des bras de compression de côté, liés sur les côtés au châssis pour appliquer une pression correctrice pour la scoliose et **en ce que** chacun des supports centraux (2) comprend :
- une colonne télescopique (9), qui est essentiellement verticale et mobile longitudinalement dans les directions vers le haut et vers le bas, ladite colonne télescopique (9) ayant une extrémité inférieure intégralement liée au châssis (1), et ;
- un plateau (10) lié à une extrémité supérieure de la colonne télescopique (9), pour supporter l'utilisateur.

2. Dispositif pour la fabrication d'un corset de traitement de scoliose selon la revendication 1, **caractérisé en ce que** la colonne télescopique (9) a un entraînement de type hydraulique.

3. Dispositif pour la fabrication d'un corset de traitement de scoliose selon la revendication 1, **caractérisé en ce que** chacun des bras de compression (3) comprend à son tour :
- une vis (12) qui est mobile transversalement par rapport au châssis (1), et
- un accouplement de type barre plate (13) lié à une extrémité libre de la vis (12), destiné à être en contact avec le torse de l'utilisateur.

4. Dispositif pour la fabrication d'un corset de traitement de scoliose selon la revendication 2, **caractérisé en ce que** l'accouplement de type barre plate (13) a une géométrie courbe pour s'adapter à l'anatomie de l'utilisateur.

5. Dispositif pour la fabrication d'un corset de traitement de scoliose selon la revendication 1, **caractérisé en ce que** l'élément mécanique de traction cervicale (4) comprend :
- un appui-tête (14) lié au châssis (1) pour supporter la tête de l'utilisateur,
- des éléments de fixation supplémentaires (15) liés à l'appui-tête (14) pour immobiliser la tête,
- un câble (16) lié intégralement au niveau d'une extrémité de l'appui-tête (14),
- un mécanisme de traction (17) lié intégralement à l'autre extrémité du câble (16) pour exécuter une force de traction, et
- un contrôleur (18) lié au mécanisme de traction (17) pour déterminer la force de traction appliquée, et par conséquent l'allongement cervical exercé chez l'utilisateur.

6. Dispositif pour la fabrication d'un corset de traitement de scoliose selon l'une quelconque des revendications précédentes, **caractérisé en ce que** les bras de compression de côté (3) comprennent une extrémité distale où sont agencés des accouplements configurés pour être adaptés à la région postéro-latérale du torse du patient.

7. Dispositif pour la fabrication d'un corset de traitement de scoliose selon l'une quelconque des revendications précédentes, **caractérisé en ce que** chacun des bras de compression de côté (3) peut être entraîné indépendamment par rapport aux autres.
